(19) Europäisches Patentamt
European Patent Office
Office européen des brevets

(11) EP 1 737 492 B1

(12) **EUROPÄISCHE PATENTSCHRIFT**

(45) Veröffentlichungstag und Bekanntmachung des Hinweises auf die Patenterteilung:
**12.12.2012 Patentblatt 2012/50**

(21) Anmeldenummer: **05731056.7**

(22) Anmeldetag: **15.04.2005**

(51) Int Cl.:
***A61K 41/00*** $^{(2006.01)}$ ***A61L 2/10*** $^{(2006.01)}$
***A61L 2/00*** $^{(2006.01)}$

(86) Internationale Anmeldenummer:
**PCT/EP2005/004032**

(87) Internationale Veröffentlichungsnummer:
**WO 2005/099757 (27.10.2005 Gazette 2005/43)**

(54) **AUFBEREITUNG FÜR PHOTODYNAMISCHE BEKÄMPFUNG VON MIKROORGANISMEN UND VERWENDUNG DER AUFBEREITUNG**

PREPARATION FOR THE PHOTODYNAMIC CONTROL OF MICRO-ORGANISMS AND USE OF SAID PREPARATION

PREPARATION DESTINEE A LA LUTTE PHOTODYNAMIQUE CONTRE DES MICRO-ORGANISMES ET UTILISATION

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **16.04.2004 DE 102004019247**
**27.05.2004 PCT/EP2004/005719**

(43) Veröffentlichungstag der Anmeldung:
**03.01.2007 Patentblatt 2007/01**

(73) Patentinhaber: **bredent medical GmbH & Co. KG**
**89250 Senden (DE)**

(72) Erfinder:
- **VIZETHUM, Freimut**
  **68199 Mannheim (DE)**
- **SCHÜTZE, Reinhold**
  **A-4800 Attnang-Puchheim (AT)**

(74) Vertreter: **Schmitt, Meinrad**
**Reble & Klose**
**Rechts- und Patentanwälte**
**Konrad-Zuse-Ring 32**
**68163 Mannheim (DE)**

(56) Entgegenhaltungen:
**WO-A-93/21992 WO-A-94/28120**
**WO-A-96/36704 WO-A-02/096471**
**WO-A-03/066109**

- **KOMERIK N; WILSON M: "Factors influencing the susceptibility of Gram-negative bacteria to toluidine blue O-mediated lethal photosensitization", JOURNAL OF APPLIED MICROBIOLOGY, vol. 92, 2002, pages 618-623,**
- **GROSS SHIMON ET AL: "Protein-A-mediated targeting of bacteriochlorophyll-IgG to Staphylococcus aureus: A model for enhanced site-specific photocytotoxicity", PHOTOCHEMISTRY AND PHOTOBIOLOGY, vol. 66, no. 6, December 1997 (1997-12), pages 872-878, XP007911288, ISSN: 0031-8655**
- **M TEICHERT ET AL: 'Treatment of oral candidiasis with methylene blue-mediated photodynamic therapy in an immunodeficient murine model1' ORAL SURGERY, ORAL MEDICINE, ORAL PATHOLOGY, ORAL RADIOLOGY & ENDODONTICS Bd. 93, Nr. 2, 01 Februar 2002, Seiten 155 - 160, XP055015573 ISSN: 1079-2104**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 737 492 B1

## Beschreibung

Die Erfindung bezieht sich die Verwendung eine Aufbereitung für die photodynamische Bekämpfung von Mikroorganismen gemäß den im Oberbegriff des Patentanspruchs 1 angegebenen Merkmalen.

Aus der WO 94/28120 A ist eine derartige Aufbereitung für die photodynamische Beaufweisenden Photosensitizer bekannt, bei dessen Bestrahlung mittels Licht Singulettsauerstoff gebildet wird. Mittels des Farbstoffs sind die zu bekämpfenden Mikroorganismen markierbar. Die Aufbereitung enthält ferner einen Wirkstoff zur Stärkung oder Schwächung der oxydativen Wirkung des Sauerstoffs, wie beispielsweise Glutathion, Mannitol oder Enzyme.

Weiterhin sind aus der WO 96/36704 A Aufbereitungen bekannt, welche zusätzlich zum Photosensitizer einen Wirkstoff zur Stärkung oder Schwächung der oxydativen Wirkung des Sauerstoffs enthalten, wie Glutathion oder Vitamin E. Auch aus der WO 96/36704 A und der WO 02/096471 A sind Aufbereitungen mit einem Photosensitizer bekannt, welche zur Stärkung oder Schwächung der oxydativen Wirkung des Sauerstoffs beispielsweise Glutathion oder Ascorbinsäure enthalten. Des Weiteren sind in der WO 03/066109 A Verfahren und Aufbereitungen zur Sterilisation der Mundhöhle beschrieben, wobei ein Photosensitizer und optional Antioxidantien vorgesehen sind. Zudem sind aus der WO 01/87416 A1 eine Anordnung und ein Verfahren zur Reduktion bzw. Zerstörung von Mikroorganismen, wie Bakterien, unter Einsatz einer lichtaktivierbaren Substanz, nach der photodynamischen Therapie (PDT) bekannt.

Mit Hilfe der lichtaktivierbaren Substanz, insbesondere eines Farbstoffes, werden die Mikroorganismen, sensibilisiert und/oder angefärbt und nach Bestrahlung mit Licht geeigneter Wellenlänge und Energiedichte abgetötet. Das Wirkungsprinzip der POT beruht, nach der selektiven Einwirkung und/oder Anfärbung der Mikroorganismen auf der physikalischen Wirkung der Energieübertragung auf die lichtaktivierbare Substanz, welche auch als Photosensitizer oder Photosensibilisator bezeichnet wird. Von dort kann die Energie für Reaktionen an der Zellmembran zur Verfügung gestellt werden. Die mittels eines Bestrahlungsgeräts, insbesondere eines Lasergeräts, erzeugte Energie wird damit auf die Mikroorganismen konzentriert und die Gleichgewichtslage von Reaktionen, welche auch im nicht belichteten Zustand im "normalen" Milieu ablaufen, werden verschoben und in der Folge werden die Mikroorganismen zerstört.

Ferner ist aus der EP 0 637 976 B1 die Verwendung einer lichtsensibilisierenden Substanz oder Verbindung bzw. eines Photosensitizers oder Photosensibilisators (PS) bei der Herstellung eines Medikamentes zur Verwendung bei der Desinfizierung oder Sterilisierung von Geweben der Mundhöhle oder einer Wunde oder Läsion in der Mundhöhle durch Zerstören von mit einer Erkrankung verbundenen Mikroben in einer periodontalen Tasche, in der Region zwischen dem Zahn und dem Zahnfleisch bekannt. Hierbei erfolgt eine Kontaktierung der Gewebe, der Wunde oder der Läsion mit dem Photosensitizer, wobei die mit einer Erkrankung verbundenen Mikroben den Photosensitizer aufnehmen. Es wird eine Bestrahlung der Gewebe, der Wunde oder der Läsion mit Laserlicht bei einer durch den Photosensitizer absorbierenden Wellenlänge durchgeführt. Die Keimreduktion dieser kombinierten Farbstoff- und Laserbehandlung wird für verschiedene Keime und Photosensitizer in Form von Lösungen mit unter anderem Methylenblau und Toluidinblau in unterschiedlichen, recht geringen Konzentrationen beschrieben, und zwar von 0,01 bis 0,00125 % (Gewicht pro Volumen), wobei ferner der Einfluß der applizierten Energiedichte aufgezeigt wird. Als Lichtquellen werden HeNe-Laser mit einer Wellenlänge von 634 nm und einer Leistung von 7,3 mW sowie GaAs-Laser mit einer Wellenlänge von 660 nm und einer Leistung von 11 mW verwendet.

KOMERIK N and WILSON M: "Factors influencing the susceptibility of Gram-negative bacteria to toluidine blue O-mediated lethal photosensitization" JOURNAL OF APPLIED MICROBIOLOGY, Bd. 92, 2002,Seiten 618-623 beschreiben den Einfluss von Speichel und dem pH-Wert der Lösung, in der Bakterien suspendiert wurden, auf die Wirksamkeit von Toluidin Blau O-herbeigeführter Photosensitivierung.

Die Photodynamische Therapie ist ein photochemisches Verfahren, das bisher vor allem in der Krebstherapie Anwendung fand. Unter dem Begriff "photodynamische Therapie" versteht man allgemein die lichtinduzierte Inaktivierung von Zellen, Mikroorganismen oder Molekülen.

Eine Modifikation dieses Prinzip findet heute in der Bekämpfung von Mikroorganismen Anwendung - die "Antimikrobielle Photodynamische Therapie" (APT). Dabei ist das Ziel nicht die Vernichtung körpereigener (Tumor) - Zellen, sondern die gezielte Bekämpfung lokaler Infektionen, die Bekämpfung von Mikroorganismen. Das Wirkungsprinzip der APT beruht auf der selektiven Anfärbung der Mikroorganismen im Biofilm durch einen sogenannten Photosensitizer und der Zerstörung der Keime durch Belichtung mit einem geeigneten, auf den Photosensitizer abgestimmten Laser.

Als Photosensitizer können Substanzen, zum Beispiel Thiazinfarbstoffe verwendet werden, welche in der Lage sind, Licht geeigneter Wellenlänge aufzunehmen und dadurch in den angeregten, sogenannten Triplettzustand übergehen.

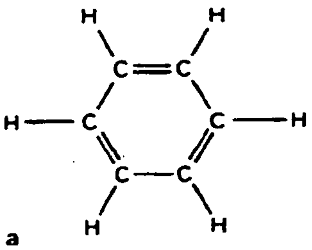
a

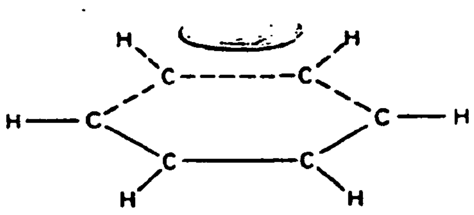
b

Aromatische Kohlenstoffringe als Grundbausteine chromophorer Moleküle

Systemisch anwendbare Photosensitizer werden vor allem in der Krebstherapie angewandt. Diese richtet sich gegen körpereigene Zellen, werden systemisch nach bestimmten pharmakologischen Grundsätzen gegeben, um sie im Tumor anzureichern und anschließend durch Bestrahlung mit Licht zu aktivieren. Diese Anwendung unterscheidet sich prinzipiell von der zur Bekämpfung oberflächlicher Infektionen.

Damit eine Substanz als effektiver Photosensitizer für diese klinische Anwendung eingesetzt werden kann, sind eine Reihe von Voraussetzungen zu erfüllen. Die Wirksubstanz sollte:

1. Nichttoxisch sein
2. Geeignete Penetrationseigenschaften und hohe bakterielle Affinität besitzen
3. Geeignete spektrale Eigenschaften aufweisen
4. Einfach und sicher zu applizieren sein
5. Vollständige Benetzung des Therapiebereiches bewirken
6. Geeignete Viskosität , möglichst thixotrope Eigenschaften besitzen
7. Auch auf offenen Wunden anwendbar sein
8. Möglichst schmerzfreie Anwendung zulassen
9. Hohe Triplettquantenausbeute (hohe Singulettsauerstoffproduktion) erreichen
10. Zeitstabil sein
11. Zugelassen sein nach Arzneimittel - oder Medizinproduktegesetz

Photosensitizer können Licht (Energie) in ihre Struktur aufnehmen und diese dann als chemische Energie für weitere Reaktionen wieder zur Verfügung stellen. Aus den, in den der Umgebung der angeregten Photosensitizermoleküle vorkommenden Sauerstoffmolekülen, wird dann eine spezielle angeregte und sehr reaktive Form gebildet, sogenannter Singulettsauerstoff.

Dabei laufen prinzipiell und vereinfacht folgende Reaktionen ab:

| | **Ausgangsprodukt** | **→ Reaktionsprodukt** | **Lebensdauer (sec)** |
|---|---|---|---|
| Gleichung 1: | $^1$Sensitizer + Licht (h*v) | → $^3$Sensitizer $^{angeregt}$ | $10^{-9}$ |
| Gleichung 2: | $^3$Sensitizer $^{angeregt}$ | → $^3$Sensitizer $^{metastabil}$ | $>10^{-8}$ |
| Gleichung 3: | $^3$Sensitizer $^{metastabil}$ $+^3O_2$ | → $^1$Sensitizer + $^1O_2$ | $10^{-6}$-$10^{-3}$ |

Unter Einfluss von geeignetem Licht und Sauerstoff reagieren die photosensibilisierenden Substanzen nach den angegebenen Reaktionswegen. Die bei der antimikrobiellen photodynamischen Therapie eingestrahlten Photonen h□regen den an den Mikroorganismen angekoppelten Photosensibilisator ($^1$Sens) aufgrund seines hohen Absorptionsvermögens an (Gleichung 1). Innerhalb von Nanosekunden geht das angeregte Sensibilisatormolekül gemäß Gleichung (2) in einen metastabilen Triplettzustand mit längerer Lebensdauer über. Der Aufenthalt im Triplettzustand (im Mikrosekundenbereich) dauert im Vergleich zu anderen Anregungszuständen (im Piko- und Nanosekundenbereich) relativ lange an und bietet deshalb ideale Voraussetzungen als Ausgangspunkt für photochemische Reaktionen, wie z. B. die Anregungsenergie auf ein Sauerstoffmolekül zu übertragen. Der angeregte Sauerstoff nimmt den energetisch höheren Singulettzustand mit einer relativ längeren Lebensdauer an (Gleichung 3).

Das Grundprinzip des antimikrobiellen photodynamischen Effekts basiert somit auf der lokalen Bildung von Singulettsauerstoff und in der Folge von reaktiven Radikalen. Dieser aktivierte Singulettsauerstoff löst Oxidationen von Molekülen vor allem in der Zellwand der Mikroorganismen und damit eine Zytolyse aus. Nach Abschalten der Energiezufuhr kommt aufgrund der sehr kurzen Lebensdauer der aktivierten Moleküle der Prozess sehr schnell - im Bereich von Sekundenbruchteilen zum Stillstand. Nun verbraucht sich der Photosensitizer dann bei den aufgebrachten Energiedosen nicht, wenn diese in einem Bereich von 3 J/cm2 bis 6 J/cm2 liegen, da in diesem Bereich das sog.Photobleaching

-die Zerstörung des Photosensitizermoleküls durch Licht - nicht festzustellen ist, sondern verbleibt im oder am Gewebe. Dabei können zum Beispiel dem Sonnenlicht ausgesetzte, behandelte Areale in unerwünschter Weise weiterreagieren und Nebenwirkungen verursachen.

Hiervon ausgehend liegt der Erfindung die Aufgabe zugrunde, die Aufbereitung dahingehend weiterzubilden, dass eine verbesserte Bekämpfung von Mikroorganismen erreicht wird.

Die Lösung dieser Aufgabe erfolgt gemäß der im Patentanspruch 1 angegebenen Merkmale.

Mittels der erfindungsgemäß vorgeschlagenen Aufbereitung, welche in flüssiger oder pastöser Form zur Markierung von Bakterien mit einem Farbstoff dient, wird in Verbindung mit der Aktivieren oder Desaktivierung dieses Farbstoffes im Hinblick auf seine Lichtabsorption, ein Verstärken oder Schwächen der oxidativen Wirkung entstehenden Singulettsauerstoffes durch chemisch Manipulation des Farbstoffes oder seiner Nanoumgebung erreicht.

Erfindungsgemäß wird davon ausgegangen, dass Photosensitizer wie zum Beispiel Phenotiazinfarbstoffe, ohne dass insoweit eine Beschränkung erfolgt, je nach anwesender Nanoumgebung in verschiedene strukturell ähnliche Formen reagieren können, wie nachfolgend für Methylenblau ersichtlich:

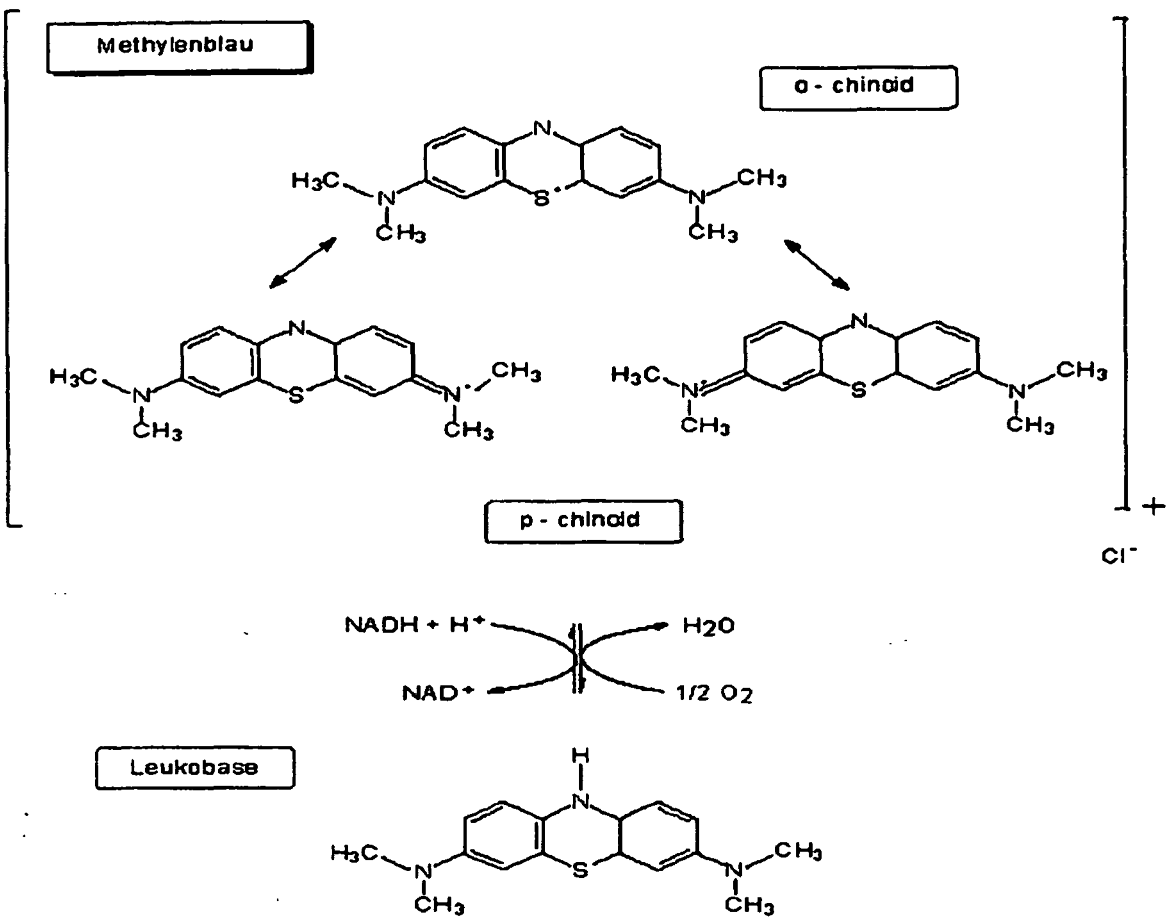


Die Leukoform des Methylenblaues ist zum Beispiel nicht photoaktiv und farblos, während die o- und p- chinoiden Formen geeignete Photosensitizer sind.

Durch die gezielte Änderung der Nanoumgebung der Moleküle kann somit der Effekt gezielt ein und ausgeschaltet werden, indem durch Protonierung durch chemische Protonendonatoren die adsorbierten Photosensitizermoleküle ein oder ausgeschaltet werden können.

Die erfindungsgemäße Aufbereitung enthält Farbstoffe, die durch einfache chemische Reaktionen reversibel aus der aktiven in die inaktive Form und umgekehrt gebracht werden können. Nach der Therapie liegen sie im Körper als unschädliche Moleküle vor und können abgebaut und ausgeschieden werden. Andererseits können auch die noch vorhandenen ggf. nach einer Zeit x (x=Stunden oder Tage) durch gezielte Änderung der Nanoumgebung wieder "aktiviert" werden.

Nachfolgend werden besondere zum "Schalten" geeignete potentielle "Schalt" - Wirkstoffe und ihre generelle Bedeutung im Stoffwechsel als Reduktionsmittel erläutert, ohne dass insoweit eine Beschränkung erfolgt.

1. Glutathion

Glutathion in reduzierter (GSH) und oxidierter Form (GSSG) besitzen folgende Strukturen:

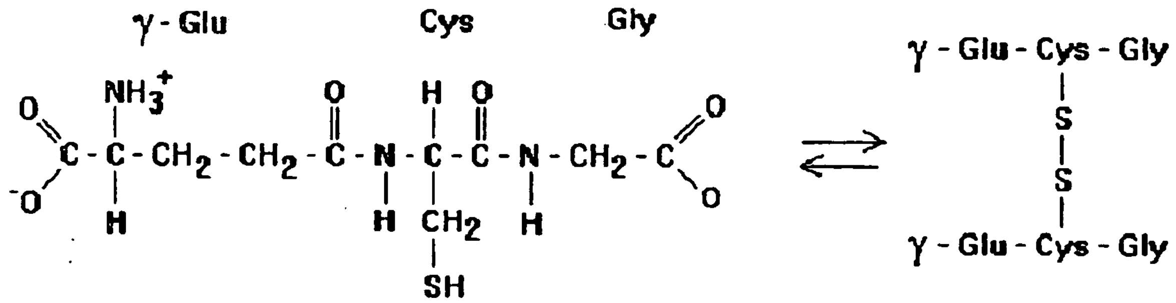

Glutathion ist ein Aminosäurederivat, das im Stoffwechsel der Zellen eine unterschiedlich wichtige Rolle spielt und in hoher Konzentration vorhanden ist. Glutathion ist in einem Redoxkreislauf eingebunden, in dem es in oxidierter Form (GSSG) und in reduzierter Form (GSH) auftritt. Oxidiertes Glutathion besteht aus zwei durch eine Disulfidverbindung miteinander verketteten Tripeptiden, währenddessen die reduzierte Thiolform ein einzelnes Tripeptid mit einer freien Sulfhydryl-Gruppe darstellt. In diesem Redoxsystem wird die Reduktion von GSSG zu GSH durch die Glutathion-Reduktase katalysiert.Hierbei ist das Gleichgewicht stark zugunsten des reduzierten Glutathion verschoben und NADPH als Cofaktor notwendig.

In den tierischen Zellen hat Glutathion zahlreiche Schlüsselpositionen bei biochemischen Prozessen inne, wobei die Wirkung als Antioxidans wahrscheinlich sehr wichig ist. Weiterhin spielt es eine enorme Rolle im Metabolismus der Cystein-enthaltenden Proteine und ist beteiligt an der Deaktivierung toxischer, elektrophiler Agentien bei durch Schwefelverbindungen katalysierten Enzymtransporten.

Im Stoffwechsel übernimmt GSH die Aufgabe eines Sulfhydryl-Puffers, der unter anderem den Cysteinrest von Hämoglobin und andere Erythrozyten-Proteine in ihrer reduzierten Form erhalten soll. Untersuchungen ergaben, dass für die natürliche Struktur der Erythrozyten reduziertes Glutathion unentbehrlich ist, da schon ein geringfügig kleinerer Spiegel an GSH eine höhere Affinität der Blutkörperchen zur Hämolyse als im Normalfall erkennen lässt. Ebenfalls bedeutend ist der Glutathion-Redoxzyklus für den Erhalt der normalen mitochondralen Funktion, wobei der GSH-Spiegel hier mit der Aktivität der Carnitin-Acylcarnitin-Translokase korreliert, die dadurch zu einem Indikator für die Glutathion-Präsenz in den Zellen wird.

Bei der im aeroben Leben unweigerlich auftretenden Produktion von Nebenprodukten, wie Hydrogen-Peroxiden und natürlichen Peroxiden besitzt Glutathion eine wichtige Entgiftungsfunktion. Hierbei ist Glutathion in ein spezifisches Scavenger-System der Zellen eingebunden, das den zahlreiche Veränderungen provozierenden Sauerstoffradikalen entgegenwirken soll. So werden beispielsweise Wasserstoffperoxid und Lipidperoxide im Glutathionzyklus in einer durch Glutathion-Peroxidase katalysierten Reaktion mit reduziertem Glutathion unter Bildung von Wasser und Sauerstoff metabolisiert, was in einer großen Anzahl von Zellen passiert. Das somit gebildete oxidierte Dimer von Glutathion (GSSG) wird dann nachfolgend im Redoxkreislauf wieder zu GSH reduziert.

Die unterstützende Wirkung von reduziertem Glutathion und Glutathion-Peroxidase beim intrazellulären Schutz gegen Schädigung durch diese reaktiven Spezies konnte in zahlreichen Studien bestätigt werden.

Zu schweren pathophysiologischen Konsequenzen kann es kommen, wenn eine zu große Anzahl von Oxidantien die Kapazität des Glutathion-Metabolismus übertrifft, woraus dann oxidativer Streß resultiert. Die dabei nicht entgifteten Oxidantien können dann Strukturproteine, Enzyme, Membranlipide und Nukleinsäuren angreifen und somit entscheidend die Zellfunktion beeinträchtigen.

Zu einer Behinderung der ATP-Synthese durch nicht entgiftete Peroxide kann es auf mitochondrialem oder glykolytischem Wege kommen. Eine intrazelluläre Anhäufung von GSSG, welches im Übermaß auch selbst Schaden (Reaktionen mit freien Sulfhydrylgruppen von Proteinen) anrichten kann, wird durch die Funktion ATP-abhängiger, Carriervermittelter Mechanismen stark vermindert.

Die hier im Vordergrund stehende Bedeutung des Glutathions als Protektor geht auf einen ausgebildeten Schutzmechanismus der Zelle zurück, der unter Zuwendung von Glutathion und anderen Reduktionsäquivalenten das Entstehen von Peroxidbildungen (an Fettsäureketten) verhindert. Ähnlich wie die Zellen anderer Organe sind natürlich auch die Myozyten mit einem solchen antioxidativen Enzymsystem ausgerüstet, welches außer dem Glutathion- Redoxsystem noch Superoxid-Dismutase (SOD) und Katalase enthält, um sich vor Schädigungen durch reaktive Verbindungen zu schützen. Allein die Fähigkeit von Glutathion als Elektronendonator Wasserstoffatome von seiner Thiol-Gruppe zu spenden, um damit meistens kohlenstoff-, sauerstoff- oder nitratbeladene Radikale zu binden und damit zu deaktivieren, macht die Wirksamkeit als Antioxidans aus.

Da sich Glutathion auch im Herzen als wichtige antioxidative Substanz und damit als Protektor erwiesen hat, gibt es zu dem Problem der möglichen therapeutischen Nutzbarkeit von exogener Glutathion-Zufuhr bei Herzinfarkt umfangreiche wissenschaftliche Untersuchungen.

Ferner wurde die Zweckmäßigkeit einer externen Glutathion-Zufuhr untersucht. Hier wurde zuerst an vorbehandelten Versuchstieren ("Yorkshire pigs") das myokardiale Glutathion durch einen potenten Glutathion-Synthesehemmer erschöpft, um dann im Vergleich mit unbehandelten Tieren das Ausmaß der Schädigungen nach oxidativen Stress feststellen zu können. Durch eine intravenöse Glutathion-Administration konnte hier ein positiver Effekt erzielt werden, da mit dieser externen Glutathion-Zufuhr der myokardiale Glutathion-Gehalt während der Ischämie anstieg und die reperfusionsbedingten Schäden bzw. das myokardiale Infarktausmaß reduziert werden konnten. Verschiedene andere Arbeiten an isoliert-perfundierten Herzmodellen haben ebenfalls gezeigt, daß eine Anreicherung des Perfusats mit GSH eine deutlich bessere Erholung der Ventrikelfunktion nach Ischämie und Reperfusion mit sich bringt. Eine verbesserte Funktion beruht jedoch weniger auf einem Ansteigen des intrazellulären GSH-Spiegel (GSH kann nicht effektiv in die Zellen transportiert werden) als vielmehr auf noch unergründeten Effekten des extrazellulären Glutathions. Nach Versuchen an einem Modell hypoxisch geschädigter renaler Tubuli wurde schlussgefolgtert, dass der cytoprotektive Effekt des exogenen Glutathion wahrscheinlich auf Glycin zurückzuführen ist, welches durch Abbau aus GSH entsteht.

Auch sei auf Glycin verwiesen, welches sich im Glutathion-erschöpften Herzen vermutlich nach Abbau reduzierter Myozyten bildet. Glycin ist nicht unbedeutend an der Detoxifikation von Acyl-CoA beteiligt, einem amphipathischen Molekül mit grenzflächenaktiven Eigenschaften, das sich besonders während myokardialer Ischämie anhäuft. Somit ist neben der "Schaltfunktion" des Gluthations im Hinblick auf die erfindungsgemäße Protonierung des Photosensitizers auch eine protektive Wirkung gegen Schäden durch die Wirkung des Singulettsauerstoffs auf das lokale Gewebe gegeben.

2. Ascorbinsäure

Ascorbinsäure besitzt folgende Strukturen:

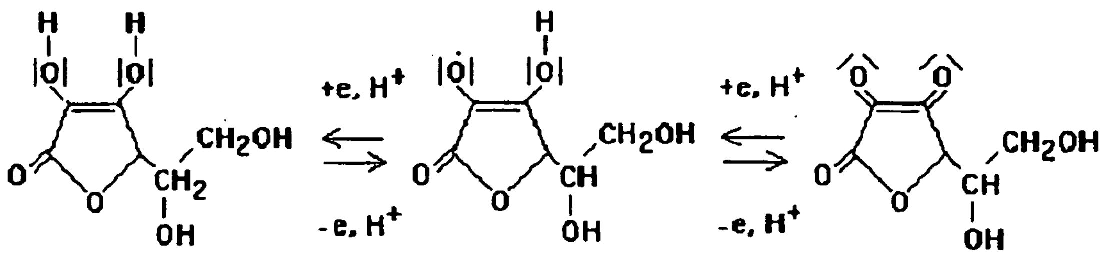


L-Ascorbinsäure L-Semihydroascorbinsäure L-Dehydroascorbinsäure

Ascorbinsäure als Protonen-Donator und -Akzeptor

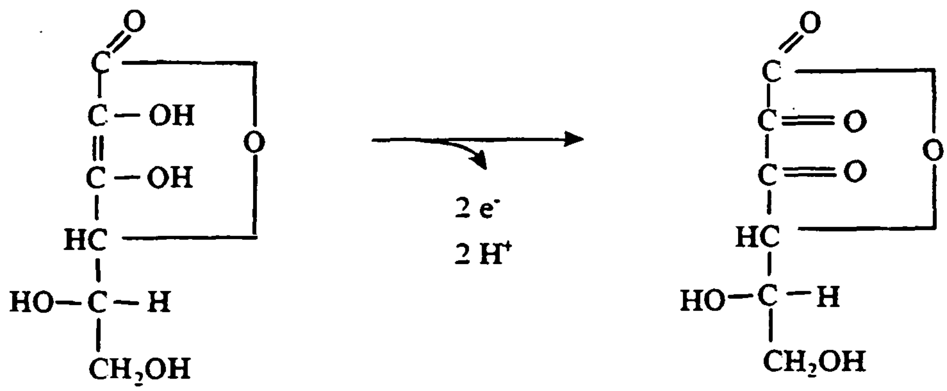


L-Ascorbinsäure (Endiolstruktur) — Dehydro-Ascorbinsäure (Diketostruktur)

Strukturell ist Ascorbinsäure ein mit Glucose und anderen Hexosen verwandtes sechsgliedriges Kohlenstoff-Ketofacton und wasserlösliches Vitamin. Im Körper wird es reversibel zu Dehydroascorbinsäure oxidiert, wirkt also im Rahmen eines Redoxgleichgewichts als Elektronendonator und Elektronenakzeptor, worauf auch die biologische Hauptwirkung beruht. Die bedeutungsvollen Redoxvorgänge laufen zwischen L-Ascorbinsäure (wirkt als 1- Elektronendonator) und der radikalischen L-Semihydroascorbinsäure ab.

Neben zahlreichen anderen Aufgaben, die Ascorbinsäure im Stoffwechsel zu bewältigen hat, steht für uns hier die Bedeutung als endogener "Scavenger" im Vordergrund. Diese Funktion als Radikalfänger geht von spezifischen Scavengersystemen aus, mit denen die Zellen ausgestattet sind, um sich vor Sauerstoffradikalen und anderen toxischen Saugerstoffmetaboliten zu schützen, die ganz eindeutig zu reversiblen und irreversiblen Gewebsschäden, unter anderem auch am Myokard führen können. Die freien Radikale kennen hierbei verschiedene Schädigungsmuster, wobei die Peroxidation von Membranphospholipiden und die Oxidation von Sulfhydrylverbindungen sicherlich am bedeutungsvollsten sind. Während es im Rahmen der Phospholipidperoxidation zur Entstehung von verschiedenen Radikalspezies, wie Lipidradikale, Lipidalkoxylradikale, Lipidperoxide und Lipidhydroperoxide kommt, werden bei der Oxidation von Sulfhydrylverbindungen essentielle Membrantransportproteine und Enzyme inaktiviert, was wiederum zur Anhäufung bestimmter Elektrolyte und damit zur Zellschädigung führt. Mit Hilfe der Antioxidantien, die in den endogen Schutzsystemen eingebunden sind (neben Ascorbinsäure auch Vitamin E und alpha-Tocopherol), versuchen die Zellen die durch eine explosions-artige Radikalproduktion ausgelösten und fortlaufenden Kettenreaktionen einzudämmen bzw. zu beenden.

Ascorbinsäure gehört zu den am stärksten reduzierenden Agentien im biologischen Milieu.

Durch Protonierung des Photosensitizers und Eindämmung der Radikalkettenreaktion kann die photodynamische Wirkung gesteuert werden.

3 Hydrochinon > Chinon

Chinone wirken an Redoxreaktionen in Mitochondrien (Atmungskette) und Chloroplasten (Photosynthese) mit. Man unterscheidet zwischen den Ubichinonen und den Plastochinonen, die sich durch unterschiedliche Seitenkettenreste am Chinonring auszeichnen. Ein Ubichinon nimmt als "Koenzym Q" eine Schlüsselstellung als primärer Elektronenakzeptor im Photosystem II der Photosynthese ein.

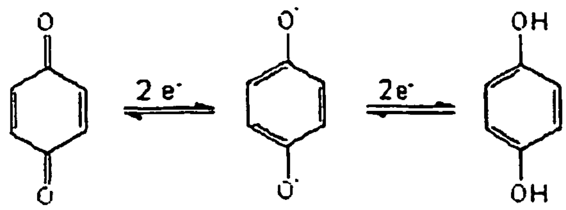


Chinon (oxydiert) Hydrochinon (reduziert)

Als Reduktionsmittel oder Protonendonator wie zum Beispiel auch bei der photographischen Entwicklung angewand, kann auch das Hydrochinon, abgekürzt $H_2Q$ verwendet werden

$$H_2Q + 2\,Ag^+ \xrightarrow{Ag} 2\,H^+ + 2\,Ag + Q$$

4 Alkohole und Aldehyde

Als Protonenspender können auch ein oder mehrwertige Alkohole dienen, die zu Aldehyden dehydriert werden. Z.B. Propanol oder Glyzerin.

2.5 0,05 bis 3% ige $Na_2S_2O_4$ - Lösung als Reduktionsmittel

6 Enzymatische Steuerung: Verstärkt und gesteuert kann der Effekt der Protonierung und Deprotonierung des Photosensitizers auch durch geeignete Enzyme werden wie zum Beispiel durch Xanthin - Dehydrogenase Xanthin - Dehydrogenase ist ein Enzym mit relativ geringer Substratspezifität. Es überträgt Wasserstoff von z. B. Formaldehyd oder Acetaldehyd auf einen geeigneten Akzeptor. Dies ist zum Beispiel beim oben erwähneten Methylenblau möglich. Durch Urethan ist wiederum das Enzym Xanthindehydrogenase hemmbar.

Ein ähnliches Reaktionsverhalten ist über das Enzymsystem Succinat- Dehydrogenase erreichbar, das den Schritt Succinat > Fumarat katalysiert - eine Reaktion, die im Citratzyklus vorkomt. Sie ist durch eine Reihe, dem Succinat ähnlicher Stoffe kompetitiv hemmbar.

Der bekannteste und häufigste Wasserstoffakzeptor ist das Nicotinamidadenindinukleotid (NAD):

$$NAD^+ + 2e^- + 2H^+ <> NADH + H^+$$

oder z.B.

$$NAD^+ + R\text{-}CHOH\text{-}R' <> NADH + H^+ + R\text{-}CO\text{-}R'$$

Eines der Protonen wird vom NAD+ direkt an den Nicotinamidring gebunden, das andere verbleibt in Lösung.

NAD+ ist ein Koenzym - es wirkt nie alleine, sondern ausschließlich nach Bindung an ein Protein. NAD+-bindende Proteine (Enzyme) gehören in die Klasse der Dehydrogenasen. Alle katalysieren die gleiche chemische Reaktion (siehe oben), unterscheiden sich jedoch in bezug auf ihre Substratspezifität. So kennt man unter vielen anderen die Alkoholdehydrogenase, die Lactatdehydrogenase, Malatdehydrogenase, Glycerinaldehydphosphatdehydrogenase u.a.

7 Verstärkung der Singulettsauerstoffwirkung durch Veränderung des Verhältnisses Wasserstoff/Deuterium

Der bei Belichtung entstehende Singulettsauerstoff wird auch durch die in Wasser vorhandenen Wasserstoffatome rasch gelöscht. In natürlicher Umgebung kommt das chemisch gleichwertige Isotop Deuterium praktisch kaum vor. Durch Ersatz eines Teils der Wasserstoffatome durch das chemisch gleiche Deuterium wird diese Löschung deutlich verringert und damit die antibakterielle Wirkung verstärkt, da die gebundenen angeregten Photosensitizermoleküle und

die von Ihnen ausgelösten Singulettsauerstoffe in ihrer Nanoumgebung ein Deuterium - statt Wasserstoffumfeld vorfinden. Längere Lebensdauer der Singulettsauerstoffe bedeutet mehr Reaktivität gegen Bakterienmembranmoleküle. Dies kann durch Ersatz von Wasserstoff durch Deuterium im Bereich von 0 bis 100% erreicht werden.

Eine besondere Ausgestaltung der Erfindung ist auf eine Spüllösung gerichtet, mit welcher die photodynamische Bekämpfung weiter optimiert wird. Nachfolgend werden die sowohl für die vorstehend beschriebene Aufbereitung als auch für die Spüllösung geltenden Wirkungszusammenhänge beschrieben.

Die photochemische Inaktivierung von Mikroorganismen beinhaltet einen komplexen Ablauf chemischer Reaktionen. Prinzipiell sind drei Grundreaktionen darstellbar:

- Das Anfärben relevanter Mikroorganismen mit einem photochemisch aktivem Farbstoff
- Die Aktivierung des Farbstoffes mit Licht geeigneter Wellenlänge und Energiedichte
- Der Ablauf der notwendigen Inaktivierungsreaktionen (Membranoxidation durch Radikale/Singulettsauerstoff)

Für den klinischen Ablauf sind somit zwei Schritte entscheidend:

- Das Anfärben mit dem photosensibilisierendem Farbstoff
- Die Belichtung des Zielareales

Farbstoffe für die histologische Färbung setzen sich aus zwei essentiellen Bestandteilen zusammen.

1. Chromophore Gruppe (Farbstoffträger),
z. B. Azo-, Benzolverbindungen.
Eine chromophore Gruppe macht ein Chemikal noch nicht zum Farbstoff, obwohl es dem Auge farbig erscheinen kann.
Hierzu bedarf es einer zweiten Komponente, die als Auxochrom (Farbhelfer) bezeichnet wird.
2. Auxochrome Gruppen (Farbhelfer),
sind entweder saure Gruppen z. B.
R-O(-) Hydroxyl-,
R-COO(-) Karboxyl-,
R-NO2(-) Nitro- Gruppen,
oder basische Gruppen z. B.
R-NH3(+) Gruppen.

Die Art der Auxochrome bestimmt die Einteilung in saure und basische Farbstoffe. Sie liegen meist in Form der betreffenden Salze vor.

Die Theorie der Färbung (Harms) besagt, dass sich positiv geladene, basische Farbstoffe an saure Zell- und Gewebekomponenten anheften, die dann den Effekt der Basopilie zeigen. Andererseits binden negativ geladene, saure Farbstoffe an positiv geladene, also azidophile Komponenten in Zellen und Geweben.

| Gewebe | Farbstoff |
|---|---|
| Negativ geladene (-) (saure) Komponenten z. B. Nucleinsäuren, Schleime "Basophilie" | (+) Positiv geladene (basische) Farbstoffe z. B. Methylenblau, Methylengrün |
| Positiv geladene (+) (basische) Komponenten z. B. eosinophile Granula "Azodophilie" | (-) Negativ geladene (saure) Farbstoffe z. B. Eosin |

Aufbauend auf Arbeiten von Robert Koch hat Paul Ehrlich in seiner Publikation "Über das Methylenblau und seine klinisch- bakterioskopische Verwertung" Zeitschrift für klinische Medizin 1881;2;710-3 die Anwendung zur von Phenotiazinfarbstoffen zur Sichtbarmachung von Bakterien beschrieben. Färbelösungen bestehen aus gelösten Farbstoffmolekülen, welche mehr oder wenig spezifisch auf Membranen reagieren. Bakterien können in der so genannten Einfachfärbung mit Methylenblau angefärbt und nachgewiesen werden.

Die zellulären Membranen von Mikroorganismen und Zellen stellen hochspezifische strukturelle Strukturen dar. Die Interaktion mit der Umgebung werden von den lokalen Konzentrations- und Ladungsverhältnissen bestimmt. Für eine Reaktion eines Moleküls, zum Beispiel eines Farbstoffes ist ein Kontakt mit der Membran notwendig.

Diese Bakterienfärbung findet in wässrigen Lösungen von 0,1% bis 1% statt und diese liegen in der Regel bei einem pH -Wert von 3-4. Der färbende Molekülanteil, das Farbstoff-Kation lagert sich an die Wand der Bakterie mit Hilfe seiner positiven Oberflächenladung an, wobei diese Farbstoffe auch als Vitalfarbstoffe bezeichnet werden.

Die Farbreaktion hängt unter anderem auch vom pH-Wert der zu färbenden Probe ab. Ein saurer pH-Wert verstärkt die Reaktion mit dem basischen Methylenblau (blaue Färbung),

Bei der photochemischen Keiminaktivierung wird nun das angelagerte Farbstoff-Kation als "Photochemische Maschine" genutzt, die die Energie des Laserlichtes absorbiert und in chemische Energie umwandelt, welche für die Produktion von Singulettsauerstoff benutzt wird!

Die Belichtung wird vor allem durch die Wahl einer geeigneten Wellenlänge, welche dem Absorptionsmaximum des Farbstoffes nahe kommen sollte, der ausreichenden Flächen - und Energiedichte, aber vor allem auch durch die gleichmäßige räumliche Verteilung des Lichtes beeinflusst. Dies ist vor allem dann entscheidend, wenn komplexe Strukturen.mit unterschiedlichen optische Eigenschaften wie Zähne, Knochen und Schleimhaut in einem Areal behandelt werden müssen.

Überraschenderweise hat sich gezeigt, dass die Wirkung der photochemische Inaktivierung von Mikroorganismen deutlich verbessert werden kann, wenn ein dritter Schritt eingeführt wird:

Erfindungsgemäß erfolgt die Vorbereitung des Therapiegebietes nach dem Anfärben und vor dem Belichten durch Ausspülen der Farbstofflösung mit einer geeigneten Spüllösung

In einer klinischen Versuchsreihe wurden Patienten mit nachgewiesenen bakteriellen Infektionen in Zahnfleischtaschen diese mit 0,1 % -iger Lösung von Methylenblau gespült und so die dort vorhandenen Bakterien und Plaque angefärbt.

Anschließend wurden die Taschen mit einem Laser über eine Optik belichtet und danach gründlich alle Taschen mit einer wässrigen Spüllösung gespült, um möglichst alle Farbstoffreste zu entfernen.

Nach der Spülung mit der Spüllösung wurde erneut eine Probe aus den Taschen entnommen um die verbleibende bakterielle Besiedelung zu bestimmen.

Im Rahmen einer erweiterten Versuchsreihe wurden die Taschen nach sorgfältiger Spülung zur Entfernung von Farbstoffresten erneut für eine Minute mit Laser und Optik belichtet. In Tabelle 1 sind die resultierenden Keimzahlen bei zwei Patienten dargestellt.

**Tab.: 1 Vergleich der Keimzahlen bei zwei Patienten vor der Behandlung, nach der Behandlung und nach erneuter Belichtung nach Spülen**

| Bakterientyp | Patient 1 vor Behandlung | Patient 1 nach Belichtung 1 | Patient 1 nach Belichtung 2 | Patient 2 vor Behandlung | Patient 2 nach Belichtung 1 | Patient 2 nach Belichtung 2 |
|---|---|---|---|---|---|---|
| **Actinobacillus actinomycetemcom.** | <1,0 x $10^2$ | <1,0 x $10^2$ | <1,0 x $10^2$ | <2,7 x $10^3$ | <4,5 x $10^2$ | <1,0 x $10^2$ |
| **Porphyromonas gingivalis** | <2,5 x $10^2$ | <1,5 x $10^2$ | <1,5 x $10^2$ | <4,6 x $10^4$ | <1.3 x $10^3$ | <2,9 x $10^2$ |
| **Tannerella forsythensis** | <1,7 x $10^5$ | <5,7 x $10^3$ | <2,5 x $10^2$ | <6,3 x $10^5$ | <5,3 x $10^3$ | <2.5 x $10^2$ |
| **Treponema denticola** | <6,6 x $10^4$ | <6,6 x $10^3$ | <2,5 x $10^2$ | <4,7 x $10^5$ | <4,1 x $10^3$ | <2.5 x $10^2$ |
| **Fusobakterium nucleatum ssp.** | <2,6 x $10^5$ | <4,4 x $10^3$ | <2,8 x $10^2$ | <3,1 x $10^5$ | <6,2 x $10^3$ | <2,5 x $10^2$ |
| **Prevotella intermedia** | <1,0 x $10^2$ | <1,0 x $10^2$ | <1,0 x $10^2$ | <1,0 x $10^2$ | <1,0 x $10^2$ | <1,0 x $10^2$ |
| **Gesamt** | <4,2 x $10^8$ | <2,3 x $10^8$ | <2,1 x $10^7$ | <4,2 x $10^8$ | <4,8 x $10^7$ | <1,4 x $10^7$ |
| **vor Behandlung =**<br>**nach Belichtung 1 =**<br>**nach Belichtung 2 =** | Probe entnommen vor Färbereaktion<br>Probe entnommen nach Belichtung mit Farbstofflösung<br>Probe entnommen nach Belichtung nach Ausspülung des Farbstoffes | | | | | |

Dabei ist zu erkennen, dass die erneute Belichtung nach dem Ausspülen der Farbstofflösung die Wirkung der photochemischen Keimtötung überraschenderweise verstärkt.

Ein ähnlicher Effekt konnte nicht erzielt werden, indem die Probenentnahme unmittelbar nach dem Ausspülen des Farbstoffes ohne erneute Belichtung erfolgte. Dieser Effekt wurde also nicht durch das "Ausspülen" der Keime

erreicht.

Auch konnte ein vergleichbarer Effekt nicht erreicht werden durch zweimalige Belichtung nach Ausspülen. Also liegt dieser Effekt auch nicht in einer Vergrößerung der Belichtungsdosis begründet.

Mit der Spülung des Therapiebereiches mit der Spüllösung vor der Belichtung wurde somit die Wirkung der Belichtung überraschenderweise deutlich verbessert.

Eine Untersuchung wesentlicher Parameter der Spüllösung ergab, dass folgende chemische und optische Parameter des Therapiebereiches verändert wurden:

- PH-Wert
- Ionenkonzentration
- Optische Transparenz
- Proteinkonzentration

Mit der Spülung nach Anfärben der Mikroorganismen vor der Belichtung kann mit einer gezielten Einstellung optischer und chemischer Parameter die Lichteinwirkung verbessert und der möglichst ungestörten Ablauf der photochemischen Reaktionsketten gewährleistet werden.

Die Spüllösung soll besitzt wenigstens eine der folgenden Eigenschaften:

- Der pH - Wert des Therapieareales wird von der für die Färbung der Mikroorganismen günstigen Beeich von 3-4 auf den, für die Singulettsauerstoffbildung günstigen Bereich von 7 bis 9 verschoben. Die Singulettsauerstoffbildung und damit der oxidative Angriff auf die Membran von Mikroorganismen ist in Lösungen mit pH-Wert von 7-9 um bis zum 5 fachen höher als im Bereich von pH 3-4.
- Die Ionenkonzentration wird verändert und Puffersysteme geschwächt (zum Beispiel aus Blut und Speichel) Die Singulettsauerstoffbildung steigt bei ungepufferten bzw. schwach gepufferten Lösungen von pH 5-6 um das doppelte, vergleicht man stark gepufferte Lösungen desselben pH-Wertes.
- Die Extinktion der Flüssigkeit im Therapiegebiet wird verringert. Abhängig von der Konzentration der Färbelösung führt eine Schichtstärke von 200 $\mu$m bei einer 1% -igen Methylenblaulösung bereits zu einer Absorption des Lichts von 95%! Andererseits sind nur Farbstoffmoleküle im Sinne der Therapie aktiv, welche direkt elektrostatisch an die Membran von Mikroorganismen gebunden sind. Die Lebensdauer der entscheidende Reaktanden der ablaufenden photochemischen Reaktionen betragen:

    o Angeregtes Photosensitizermolekül = 1 x 10-9 sec

    o Singulettsauerstoff = 1x 10-4 bis 10-7

Damit liegt die mittlere freie Weglänge der reaktiven Singulettsauerstoffmoleküle bei ca 0,2 nm. Somit bleiben deren Reaktionsmöglichkeiten auch auf diesen Radius um das Molekül beschränkt. Die in freier Lösung verbleibenden Farbstoffmoleküle sind somit nicht nützlich im Sinne der Therapie, sondern verhindern durch die Erhöhung der Extinktion sogar den therapeutischen Effekt.

- Sie wirkt blutstillend und / oder verringert allgemein die Konzentration von Plasmaproteinen verringern, da diese ebenfalls als konkurrierende Reaktionspartner für Singulettsauerstoff und gebildete Radikale dienen könnten

Somit sind Spüllösungen auf wässriger oder nichtwässriger Basis vorgesehen, welche wenigstens eine der folgenden Eigenschaften aufweisen:

- einenpH-Wert von 7-9 besitzen,
- keine Konzentrationen von solchen Verbindungen enthalten, die als Puffersysteme dienen könnten (z. B. Phosphate, Citrate, Carbonate),
- die Extinktion des angewendeten Laserlichtes nicht erhöhen,
- die Farbstofflösung wirksam ausspülen,
- blutstillende Wirkung aufweisen.

Erfindungsgemäß wird die Aufbereitung, welche Farbstoff enthält oder ein solcher ist, zunächst in hoher Konzentration auf den zu therapierenden Bereich aufgebracht und nachfolgend wird eine Spülung mit der Spüllösung, insbesondere Wasser und/oder mit einem möglichst basischen pH-Wert durchgeführt. Nachfolgend wird die Bestrahlung mittels des Lichts des Bestrahlungsgeräts durchgeführt, wobei in bevorzugter Weise eine optimierte Zellschädigung erfolgt. Es hat sich als besonders effektiv erwiesen, die Aufbereitung zunächst in hoher Konzentration auf den zu therapierenden Bereich aufzubringen und nachfolgend eine Spülung mit der Spüllösung, insbesondere Wasser und / oder mit einem möglichst hohem Sauerstoffpartialdruck, durchzuführen und schließlich die Bestrahlung mittels des Lichts der genannten Lichtquelle durchzuführen, wobei bevorzugt eine optimierte Zellschädigung erfolgt. Des Weiteren hat es sich als besonders zweckmäßig erwiesen, dass nach dem Aufbringen der Aufbereitung in hoher Konzentration auf den zu therapierenden Bereich und ferner vor der Belichtung mittels des Lichts der Lichtquelle eine Verringerung der Menge der Aufbereitung durchgeführt wird, und zwar insbesondere durch Abwischen und/oder Abtupfen und/oder Absaugen und/oder Abblasen.

Mit der Applikation der lichtaktivierbaren Substanz wird langsam begonnen, welche mittels Spritze flächendekkend auf die infizierten Gewebsareale aufgebracht wird. Die Menge ist unbedingt so zu wählen, dass der die lichtaktivierbare Substanz die Oberfläche der infizierten Bereiche möglichst dünn benetzt. Die vollständige Benetzung von Nischen und Taschen im Gewebe ist sicherzustellen. Gegebenenfalls bei komplexer Morphologie mit dem Luftbläser vorsichtig verteilen. Die Einwirkzeit der lichtaktivierbaren Substanz beträgt mindestens 60sec. Nach Spülung für mindestens 3 sec mit gleichzeitiger Absaugung überschüssiger Lösung (unbedingt Farbstoff-Depots entfernen!) mit dem Bestrahlungsgerät belichten. Wesentlich für die keimreduzierende Wirkung und damit für das Behandlungsergebnis, ist die richtige Dosierung der Energiezufuhr, die Belichtung.

Nach der Einwirkzeit, der Aufbereitung oder Photosensitizers (PS), die mit mindestens 60 sec. angegeben ist, wird erfindungsgemäß der überschüssige PS ausgespült und gleichzeitig die Spüllösung abgesaugt. Damit werden PS und Exsudat entfernt und nur die verbleibenden Reste stehen für eine orale ingestinale Aufnahme zur Verfügung.

Tabelle Verdünnungsverhältnis nach Ausspülen und Absaugen des PS

| Spüllösung in ml | Verdünnung | Gehalt MB in % | Molar | Gehalt MB in mg/ml | mg Substanz in 0,5 ml |
|---|---|---|---|---|---|
| 0 | Ausgangslösung | 1 | 0.031 | 10 | 5 |
| 5 | 1.10 | 0,01 | 0,003 | 1 | 0,5 |
| 50 | 1:100 | 0,001 | 0,0003 | 0,01 | 0,05 |

Wie aus obiger Tabelle ersichtlich, verbleiben bei Ausspülen, Verdünnen und Absaugen des PS um den Faktor 10 noch 0,5 mg und bei Verdünnen um den Faktor 100 noch 0,05 mg Substanz. Dies würde bei hundertfacher Verdünnung (Spülen mit Wasser), unter der Annahme, dass die gesamte verbleibende Menge oral aufgenommen und resorbiert wird, bei einem Erwachsenen mit einem mittleren angenommenen Körpergewicht von 65 kg einer von 0,0008 mg pro kg Körpergewicht oder 0,08 Mikrogramm pro kg Körpergewicht entsprechen. Bei einem Kind mit einem angenommenen Gewicht von 20 kg wären dies 0,0025 Milligramm pro kg Körpergewicht, bzw. 2,5 Mikrogramm pro kg Körpergewicht.

Für die, als Antidot verwendete, 1%ige Lösung zur Injektion wird eine Tages-Maximaldosis bis 200 mg angegeben. Dies bedeutet für einen Erwachsenen eine kg Dosis pro Tag von ca. 3 mg und für ein Kind von 10 mg/Tag/kg.

In O,5ml 1%iger Lösung ist maximal 5 mg MB gelöst. Selbst wenn der Patient alles schluckt, beträgt die kg Dosis pro Tag nur ca. 80 μg und für ein Kind 250 μg. dies entspricht nur ca. 2,5% der für die Entgiftung empfohlenen Dosis ohne Berücksichtigung der o.a. therapeutisch empfohlenen Verdünnung vor Belichtung.

Ein weiterer wesentlicher Faktor, der das Ausspülen der Aufbereitung oder des Photosensitizers (PS) vor der Belichtung betrifft, ist die hohe Absorption der Aufbereitung oder des Photosensitizers (PS) im Bereich der Wellenlänge des applizierten Lichtes.

Messungen zeigen, dass ein auf dem Gewebe stehender Flüssigkeitsfilm der Aufbereitung oder des Photosensitizers von 100 μm um die effektive Energiedichte um 97% verringert. Bei weiterer Verdopplung der Schichtdicke wird jeweils, gemäß dem Lampert-Beerschen Gesetz, das Licht weiter geschwächt. Somit ist eine therapeutisch wirksame Bestrahlung bei überschüssiger Aufbereitung oder Photosensitizer nicht möglich.

Aufgrund der Spülung mit der Spüllösung, insbesondere Wasser, möglichst geringer Ionenkonzentration werden Bakterien und / oder Zellmembranen aufgrund des somit erzeugten osmotischen Druck-Gradienten gefährdet. Es sei festgehalten, dass beispielsweise eine physiologische Kochsalzlösung aufgrund der relativ hohen Ionenkonzentration ungünstig wäre. Der pH-Wert der Spüllösung wird vorteilhaft eher basisch vorgegeben. Bevorzugt wird ein pH-Wert von 7 bis 9 vorgegeben. Der Sauerstoffpartialdruck ist bevorzugt groß vorgegeben. Im Rahmen der Erfindung weist die Spüllösung, insbesondere aufbereitetes Leitungswasser, zur Spülung einen Sauerstoffpartialdruck im Bereich von 4 bis 6 mg/l auf. Zweckmäßig ist die Spüllösung angereichert mit molekularem Sauerstoff bis zu 14 mg/l. Weiterhin hat sich erfindungsgemäß eine Peroxidanreicherung als zweckmäßig erwiesen, und zwar bevorzugt als 0,5% bis 3%ige Wasserstoffperoxidlösung.

Aufgrund der vorherigen Spülung mit der Spüllösung geringen Ionenkonzentration wird bei der Bestrahlung mittels des Laserlichts eine optimierte Zellschädigung durchgeführt. Die Aufbereitung und die Kombination mit der Spüllösung ergibt erfindungsgemäß eine gezielte Änderung des pH-Wertes im Arbeitsbereich in drei Schritten:

● Färben im saueren Bereich, insbesondere bei einem pH-Wert zwischen 3 bis 5.
● Einstellen bzw. Abschalten eher im saueren Bereich, mittels der Spüllösung, insbesondere Ascorbinsäure
● Belichten im neutralen bis leicht basischen Bereich, insbesondere bei einem pH-Wert zwischen 7 bis 9.

## Patentansprüche

1. Aufbereitung in flüssiger oder pastöser Form zur Verwendung für die photodynamische Bekämpfung von Mikroorganismen in Zahnfleischtaschen, enthaltend einen farbstoffaufweisenden Photosensitizer bei dessen Bestrahlung mittels Licht Singulettsauerstoff gebildet wird, wobei mittels des Farbstoffes die Mikroorganismen markierbar sind, insbesondere enthaltend einen Wirkstoff zur Verstärkung der oxydativen Wirkung des Singulettsauerstoffes durch chemische Manipulation des Farbstoffes oder seiner Nanoumgebung in Kombination mit einer Spüllösung, mittels welcher zur Verbesserung der Wirksamkeit der Aufbereitung der Photosensitizer nach dem Anfärben der Mikroorganismen und vor der Bestrahlung ausspülbar ist, **gekennzeichnet dadurch, dass** der Photosensitizer zunächst in hoher Konzentration auf den zu therapierenden Bereich aufgebracht wird, dass nachfolgend eine Spülung mit der Spüllösung, insbesondere Wasser und/oder mit möglichst geringer Ionenkonzentration durchgeführt wird und dass schließlich die Bestrahlung mittels des Laserlichtes durchgeführt wird wobei bevorzugt eine optimierte Zellschädigung erfolgt, oder dass der Photosensitizer zunächst in hoher Konzentration auf den zu therapierenden Bereich aufgebracht wird, dass nachfolgend eine Spülung mit der Spüllösung, insbesondere Wasser und/oder möglichst basischem PH-Wert durchgeführt wird und dass schließlich die Bestrahlung mittels des Laserlichtes durchgeführt wird, wobei bevorzugt eine optimierte Zellschädigung erfolgt.

2. Aufbereitung zur Verwendung nach Anspruch 1, **dadurch gekennzeichnet, dass** die Spüllösung derart ausgebildet ist, dass durch das Ausspülen der pH-Wert des Therapieareals in einen für die Singulettsauerstoffbildung günstigen Bereich von 7 bis 9 verschoben wird und/oder die Ionenkonzentration veränderbar sind.

3. Aufbereitung zur Verwendung nach Anspruch 1 oder 2, **dadurch gekennzeichnet, dass** die Spüllösung derart ausgebildet ist, dass durch das Ausspülen Puffersysteme schwächbar sind und/oder die Extinktion der Flüssigkeit im Therapieareal verringerbar ist und/oder eine lokale blutstillende Wirkung erzielbar ist und/oder die Konzentration von Plasmaproteinen verringerbar ist.

4. Aufbereitung zur Verwendung nach einem der Ansprüche 1 bis 3, **dadurch gekennzeichnet, dass** die Spüllösung auf wässriger oder nicht wässriger Basis wenigstens eine der folgenden Eigenschaften aufweist:

   ● einen pH-Wert von 7 bis 9,
   ● keine Konzentrationen von solchen Verbindungen, welche als Puffersysteme dienen könnten,
   ● keine Erhöhung der Extinktion angewendeten Laserlichts,
   ● eine wirksame Ausspülbarkeit der Farbstofflösung,
   ● eine blutstillende Wirkung.

5. Aufbereitung zur Verwendung nach einem der Ansprüche 1 bis 4, **dadurch gekennzeichnet, dass** die Spüllösung, insbesondere aufbereitetes Leitungswasser, einen Sauerstoffpartialdruck von 4-6 mg/l aufweist und/oder mit molekularem Sauerstoff bis 14 mg/l angereichert ist und/oder dass das genannte Medium mit einem Peroxid angereichert ist, insbesondere mit einer 0,5 bis 3%igen Wasserstoffperoxidlösung.

6. Aufbereitung zur Verwendung nach einem der Ansprüche 1 bis 5, **dadurch gekennzeichnet, dass** der Farbstoff im Hinblick auf seine Lichtabsorbtion aktivierbar oder deaktivierbar ist und/oder dass durch Protonierung durch chemische Protonendonatoren die absorbierten Photosensitizermoleküle ein- oder ausschaltbar sind und/oder dass durch Protonierung des Photosensitizers und Eindämmung der Radikalkettenreaktion die photodynamische Wirkung steuerbar ist.

7. Aufbereitung zur Verwendung nach einem der Ansprüche 1 bis 6, **dadurch gekennzeichnet, dass** zumindest ein Teil der Wasserstoffatome des Wassers durch Deuterium ersetzt ist.

8. Aufbereitung zur Verwendung nach einem der Ansprüche 1 bis 7. **dadurch gekennzeichnet, dass** der Photosensitizer in einem Lösungsmittel eine Konzentration, und zwar Gewicht pro Volumen, von größer als 0,1%, bevorzugt größer als 0,5% und insbesondere im wesentlichen von 1 % aufweist und/oder dass für die Konzentration eine Obergrenze von 10%, bevorzugt 4%, insbesondere von 3%, vorgegeben ist.

9. Aufbereitung zur Verwendung nach einem der Ansprüch 1 bis 8 **dadurch gekennzeichnet, dass** die lichtaktivierbare Substanz zunächst in hoher Konzentration auf den zu therapierenden Bereich aufgebracht wird, dass nachfolgend eine Spülung mit der Spüllösung. insbesondere Wasser und/oder mit möglichst hohem Sauerstoffpartialdruck, durchgeführt wird und dass schließlich die Bestrahlung mittels des Laserlichts durchgeführt wird, wobei bevorzugt eine optimierte Zellschädigung erfolgt, oder dass die lichtaktivierbare Substanz zunächst in hoher Konzentration auf den zu therapierenden Bereich aufgebracht wird, dass nachfolgend und vor der Belichtung eine Verringerung der Farbstoffmenge durch Abwischen, Abtupfen. Absaugen oder Abblasen erfolgt.

## Claims

1. Preparation in liquid or paste form for use for the photodynamic combating of micro-organisms in gum pockets, comprising a photosensitiser containing dye which on exposure to light forms singlet oxygen, whereby the micro-organisms can be marked by means of the dye, more particularly comprising an active substance for intensifying the oxidative effect of the singlet oxygen through chemical manipulation of the dye or its nano-environment in combination with a rinsing solution with which, in order to improve the efficacy of the preparation the photosensitiser can be rinsed out after dyeing the microorganisms, **characterised in that** the photosensitiser is initially applied in a high concentration to the area to be treated, **in that** rinsing with the rinsing solution, more particularly water and/or with as low an ion concentration as possible, is then carried out, and **in that** finally irradiation with the laser light is then carried out, whereby preferably optimised cell damage takes place, or **in that** the photosensitiser is initially applied in a high concentration to the area to the treated, **in that** rising with the rinsing solution, more particularly water and/or with as base a pH value as possible is carried out, and **in that** finally irradiation with the laser light is then carried out, whereby preferably optimised cell damage takes place.

2. Preparation for use in accordance with claim 1 **characterised in that** the rinsing solution is such that through the rinsing the pH value of the treatment area is shifted into a range from 7 to 9 which is favourable for singlet oxygen formation and/or the ion concentration can be changed.

3. Preparation for use in accordance with claim 1 or 2 **characterised in that** the rinsing solution is such that through the rinsing puffer systems can be weakened and/or the extinction of the liquid in the treatment area can be reduced and/or a local haemostatic effect can be achieved and/or the concentration of plasma proteins can be reduced.

4. Preparation for use in accordance with claims 1 to 3 **characterised in that** the aqueous or non-aqueous based rinsing solution exhibits at least one of the following properties:

    - a pH value of 7 to 9,
    - no concentrations of compounds which could act as buffer systems,
    - no increase in the laser light used in the extinction
    - effective rinsing out of the dye solution
    - a haemostatic effect.

5. Preparation for use in accordance with any one of claims 1 to 4 **characterised in that** the rinsing solution, more particularly treated tap water, has an oxygen partial pressure of 4-6 mg/l and is enriched with molecular oxygen up to 14 mg/l and/or **in that** said medium is enriched with a peroxide, more particularly with a 0.5 to 3% hydrogen peroxide solution.

6. Preparation for use in accordance with any one of claims 1 to 5 **characterised in that** the dye can be activated and deactivated in terms of its light absorption and/or **in that** through protonation by way of chemical proton donors the absorbed photosensitiser molecules can be switch on or off and/or **in that** through protonation of the photosensitiser and stemming of the radical chain reaction the photodynamic effects can be controlled.

7. Preparation for use in accordance with any one of claims 1 to 6 **characterised in that** at least some of the hydrogen atoms of the water are replaced with deuterium.

8. Preparation for use in accordance with any one of claims 1 to 7 **characterised in that** in a solvent the photosensitiser has a concentration, namely a weight per volume, of more than 0.1%, preferably greater than 0.5% and more particularly essentially 1% and/or **in that** an upper limit of 10%, preferably 4%, more particularly 3% is defined for the concentration.

9. Preparation for use in accordance with any one of claims 1 to 8 **characterised in that** the light-activatable substance is applied in a high concentration to the area to be treated, **in that** rising with the rinsing solution, more particularly water and/or with as high as oxygen partial pressure as possible then takes place, and **in that** finally irradiation with the laser light is carried out, whereby preferably optimised cell damage takes place, or **in that** the light-activatable substance is initially applied to the area to be treated in a high concentration, **in that** thereafter and before irradiation the quantity of dye is reduced by wiping, dabbing, suction or blowing off.

**Revendications**

1. Préparation sous forme liquide ou pâteuse destinée à la lutte photodynamique contre des micro-organismes dans les poches parodontales, contenant un photosensibilisant présentant des colorants lors de l'irradiation à la lumière duquel de l'oxygène singulet se forme, les micro-organismes pouvant être marqués à l'aide du colorant, contenant notamment un principe actif pour renforcer l'effet oxydant de l'oxygène singulet par manipulation chimique du colorant ou de son nano-environnement en association avec une solution rinçante, à l'aide de laquelle pour améliorer l'effet de la préparation, le photosensibilisant peut se rincer après coloration des micro-organismes et avant l'irradiation, **caractérisée en ce qu'**on applique d'abord le photosensibilisant en concentration élevée sur la zone à soigner, **en ce qu'**ensuite, on procède à un rinçage à la solution rinçante, notamment de l'eau et/ou à une concentration ionique la plus faible possible et **en ce que** pour finir, on procède à l'irradiation à l'aide de la lumière à laser, alors qu'il s'effectue de préférence une détérioration cellulaire optimisée ou **en ce qu'**on applique d'abord le photosensibilisant en concentration élevée sur la zone à soigner, **en ce qu'**ensuite, on procède à un rinçage à la solution rinçante, notamment de l'eau et/ à une valeur pH la plus basique possible et **en ce que** pour finir, on procède à l'irradiation à l'aide de la lumière à laser, alors qu'il s'effectue de préférence une détérioration cellulaire optimisée.

2. Préparation destinée à être utilisée selon la revendication 1, **caractérisée en ce que** la solution rinçante est constituée de telle sorte que par le rinçage, la valeur pH de l'aire à soigner soit décalée dans une plage de 7 à 9 favorable pour la formation d'oxygène singulet et/ou **en ce que** la concentration ionique est modifiable.

3. Préparation destinée à être utilisée selon la revendication 1 ou 2, **caractérisée en ce que** la solution rinçante est constituée de telle sorte que par le rinçage, des systèmes tampons puissent être fragilisés et/ou que l'extinction du liquide puisse être réduite dans l'aire à soigner et/ou qu'un effet hémostatique local puisse être atteint et/ou que la concentration de protéines plasmatiques puisse être réduite.

4. Préparation destinée à être utilisée selon l'une quelconque des revendications 1 à 3, **caractérisée en ce que** la solution rinçante sur base aqueuse ou sur base non aqueuse présente au moins l'une des propriétés suivantes :

   - une valeur pH de 7 à 9
   - aucune concentration de composés qui pourraient faire office de systèmes tampons,
   - aucune augmentation de la lumière laser utilisée pour l'extinction
   - une possibilité de rinçage efficace de la solution colorante
   - un effet hémostatique.

5. Préparation destinée à être utilisée selon l'une quelconque des revendications 1 à 4, **caractérisée en ce que** la solution rinçante, notamment de l'eau du robinet traitée présente une pression partielle d'oxygène de 4 à 6 mg/l et/ou est enrichie jusqu'à 14 mg/l en oxygène moléculaire et/ou **en ce que** le milieu cité est enrichi avec un peroxyde, notamment avec une solution de peroxyde d'hydrogène à de 0,5 à 3 %.

6. Préparation destinée à être utilisé selon l'une quelconque des revendications 1 à 5, **caractérisée en ce que** le colorant est activable ou désactivable au niveau de son absorption de lumière et/ou **en ce que** par protonation par des donateurs protoniques chimiques, les molécules photosensibilisantes absorbantes peuvent être excitées ou inoculées et/ou en ce que par protonation du photosensibilisant et contention de la réaction des chaînes radicales, l'effet photodynamique peut être contrôlé.

7. Préparation destinée à être utilisée selon l'une quelconque des revendications 1 à 6, **caractérisée en ce qu'**au moins une partie des atomes d'hydrogène de l'eau est remplacée par du deutérium.

8. Préparation destinée à être utilisée selon l'une quelconque des revendications 1 à 7, **caractérisée en ce que** dans un solvant, le photosensibilisant présente une concentration, à savoir en poids par volume supérieure à 0,1 %, de préférence supérieure à 0,5 % et notamment sensiblement de 1 % et/ou **en ce que** pour la concentration, il est prescrit une limite supérieure de 10 %, de préférence de 4 %, notamment de 3 %.

9. Préparation destinée à être utilisée selon l'une quelconque des revendications 1 à 8, **caractérisée en ce qu'**on applique d'abord la substance photoactivable en concentration élevée sur la zone à soigner, **en ce que** par la suite, on procède à un rinçage à la solution rinçante, notamment de l'eau et/ou avec une pression partielle en oxygène la plus élevée possible et **en ce que** pour finir, on procède à l'irradiation à l'aide de la lumière à laser, alors qu'il s'effectue de préférence une détérioration cellulaire optimisée ou **en ce qu'**on applique d'abord la substance photoactivable en concentration élevée sur la zone à soigner, **en ce que** par la suite et avant l'exposition à la lumière, on réduit la quantité de colorant par essuyage, tamponnement, aspiration ou soufflage.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9428120 A
- WO 9636704 A
- WO 02096471 A
- WO 03066109 A
- WO 0187416 A1
- EP 0637976 B1


### In der Beschreibung aufgeführte Nicht-Patentliteratur


- **KOMERIK N ; WILSON M.** Factors influencing the susceptibility of Gram-negative bacteria to toluidine blue O-mediated lethal photosensitization. *JOURNAL OF APPLIED MICROBIOLOGY,* 2002, vol. 92, 618-623